# EUROPEAN PATENT SPECIFICATION

(11) **EP 2 398 767 B1**
(45) Date of publication and mention of the grant of the patent: **13.05.2015**
(21) Application number: 09788166.8
(22) Date of filing: 23.02.2009
(51) Int. Cl.: C07C 263/20, C07C 265/14

(54) **PROCESS FOR CRYSTALLIZING AND SEPARATING DIFFERENT DIISOCYANATE ISOMERS**
VERFAHREN ZUR KRISTALLISATION UND ABTRENNUNG VON VERSCHIEDENEN DIISOCYANATISOMEREN
PROCÉDÉ POUR CRISTALLISER ET SÉPARER DIFFÉRENTS ISOMÈRES DE DIISOCYANATE

(43) Date of publication of application: 28.12.2011
(73) Proprietor: GEA Niro PT B.V., 5221 EE's-Hertogenbosch (NL)
(72) Inventor: RUEMEKORF, Ray Sircy, NL-5241 JZ Rosmalen (NL); SCHOLZ, Reinhard Uwe, 47647 Kerken (DE)
(74) Representative: Jansen, Cornelis Marinus
(86) International application number: PCT/NL2009/050083
(87) International publication number: WO 2010/095927

(56) References cited:
- EP-A- 1 475 367
- WO-A-98/25680
- WO-A-98/25889
- WO-A2-2010/040675
- M. STEPANSKI ET AL.: "New Hybrid Process for Purification and Separation of MDI Isomers" POLYURETHANES CONFERENCE 2002, CONFERENCE PROCEEDINGS, SALT LAKE CITY, UT, UNITED STATES, OCT. 13-16 (2002), 2002, pages 594-600, XP008113325 cited in the application

## Description

The invention is directed to a process for the suspension crystallization of 4,4' MDI rich streams and subsequent partitioning of the resulting mother liquor containing crystals into separate product streams containing different and useful compositions of the original isomers.

### Background of the invention

Isocyanates are industrially relevant compounds containing the functional isocyanate group (-NCO). They react with compounds containing alcohol groups (-OH) to produce polyurethane polymers, which are components of flexible and rigid foams, insulation, thermoplastic elastomers, spandex fibers, industrial adhesives and polyurethane paints. The production process and polymerization paths of the various isocyantes are detailed in numerous publications such as The Kirk-Othmer Encyclopedia of Chemical Technology, 4th edition, Vol 14, pp 446-461. Diisocyanates (*viz*. hydrocarbon compounds having two -NCO groups at different positions of the molecule) are the raw materials that make up most typical polyurethane products. The most common diisocyanates used today are toluene diisocyanate (TDI) and methylene diphenyl diisocyanate (MDI).

The production process for these feed stocks generates undesirable impurities in the form of other isomers and reaction byproducts that must be separated from the final product. Distillation or vacuum distillation is typically used to separate most of the impurities. However, the isomers are generally difficult to remove due to their similarities with the main product and as a result the use of distillation for such purification is generally a capital and energy intensive process

In the case of MDI, the main isomer is 4,4' MDI with 2,4' MDI and 2,2' MDI as the main impurities. The ratio of the 4,4' MDI and 2,4' MDI isomers in the diisocyanate solution will affect the properties of the final polyurethane. Mixtures of the isomers as well as pure 4,4' MDI are commercially desirable products.

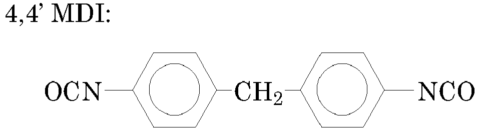

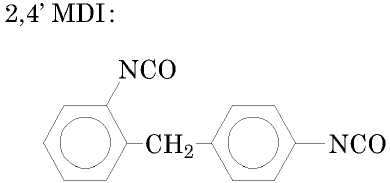

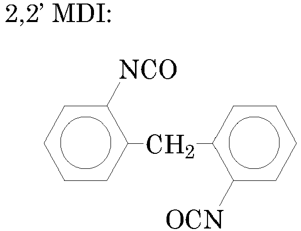

Pure 4,4' MDI is used in fibers, high performance elastomers and adhesives, while MDI mixtures with higher 2,4' MDI content are used in rigid and flexible foams. Between these two are found MDI mixtures of different compositions. These different composition result in different physical properties, such as viscosity, reactivity, stability and crystal formation. High purity MDI for textile fiber is obtained through fractional separation and requires high performance properties for the elastomeric fiber. There are various definitions for Pure MDI which all refer to a substantially pure 4,4' MDI product with an isomer purity of 95-99%. In their extensive data collection on MDI "MDI and TDI: Safety, Health and the Environment: a Source Book and Practacal Guide, 2nd ed., Wiley & Sons, 2003, ISBN 0471958123, 9780471958123" with contribution from all major MDI producers worldwide, Dennis C. Allport et al., defined Pure MDI as a substance that comprises 98% of 4,4' MDI (page 13 of 438). Pure MDI is the raw material for molded prepolymers in for instance shoe soles production, flexible adhesives, leather coatings, thermoplastic polyurethanes (TPU's), hot melt adhesives, and the like. Due to the customer's demand for different applications, a wide range of pure MDI's is produced, with specific antioxidants and modified acidity depending on the manufacturer and customer requirements.

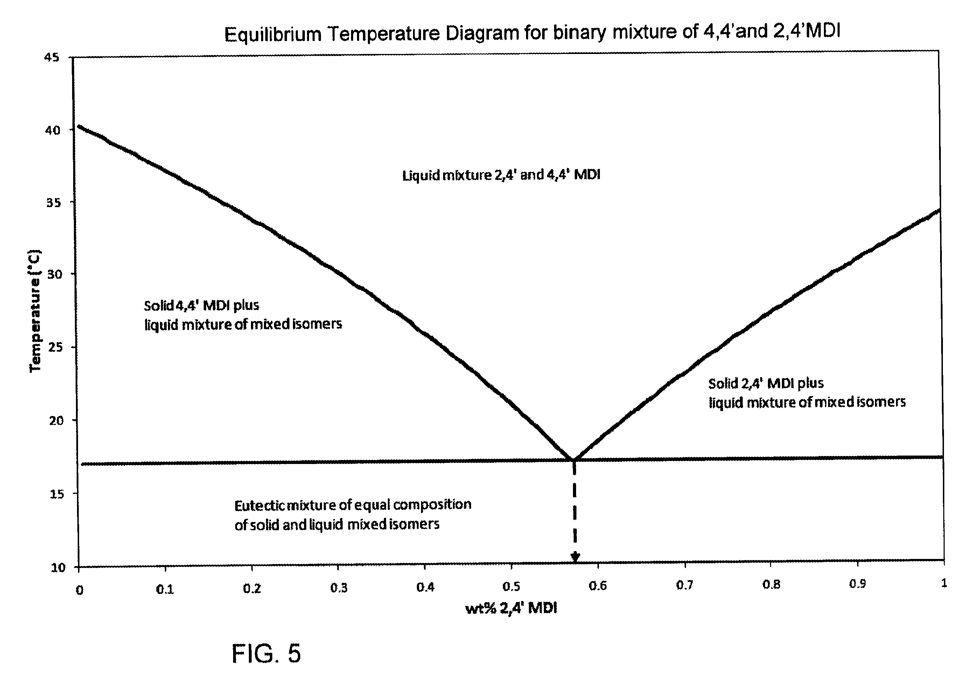

Additional background information regarding the specific chemistry involved in the production and use of TDI and MDI can be found in various works including Dennis C. Allport et al., "MDI and TDI: A Safety, Health and the Environment: a Source Book and Practacal Guide",2nd ed., Wiley & Sons, 2003, ISBN 0471958123, 9780471958123. MDI will slowly dimerize at room temperature even as a solid. Figure 5.3.1, in the above referenced book, illustrates the dimerization rate for 4,4' MDI at normal temperatures. Dimerization will become more pronounced with increasing temperature. The presence of dimer in the isocyanate solution negatively affects the properties of the final product and should be minimized. The dimerization is reversible. By converting the product to vapor the monomer can be obtained again. This requires additional handling and energy and it is therefore economically and ecologically attractive to provide a method that can achieve the desired product split of the isocyanates in a single and efficient operation that can minimize the time that the product is held in the system and allows for low process temperatures at the same time.

This is one reason why melt crystallization is typically preferred over other processes operating at higher temperatures. Arkenbout indicates in his book (Arkenbout, G. F., "Melt Crystallization Technology" Technomic, 1995 (ISBN number 1-56676-181-6), pp. 315-316) that a layer crystallization process can produce pure MDI (>99.5%) with repeated crystallization of up to 7 steps.

A combination of distillation, layer melt crystallization and thin film evaporation is used in Stepanski, M, "New Hybrid Process for Purification and Separation of MDI Isomers", Sulzer Technical Review, PU Conference 2002.

In discontinuous static and dynamic layer crystallization processes the isomer to be separated is deposited on stationary cooled surfaces, subsequently separated from the mother liquor by gravity draining of such mother liquor and then re-melted by warming up the stationary heat exchanger surfaces. Such layer crystallization processes are characterized by relatively high crystal growth rates between 10⁻⁵ m/s and 10⁻⁶ m/s which in turn result in an impure crystal product. The crystal lattice typically would still remain pure, but the crystals are grown on a cooled surface in a dendrite like structure and mother liquor containing all other isomers and the impurities gets entrapped into the multifaceted structure. It is known that such dynamic impurity inclusion effects become more pronounced with increasing viscosity. The separation efficiency of a single layer melt crystallization process can be enhanced by additional purification methods like sweating and washing: these methods offer an increase in purification efficiency at the expense of the yield. If such a layer melt crystallization process is performed in an optimized way by a person skilled in the art, the solid phase can typically be 5 to 10 times purer than the liquid melt, in case additional sweating and/or washing is applied this purification ratio can be as high as 20. Apart from the obvious economical disadvantage of the required process repetitions, such replications lead to a lower process yield and to a higher energy consumption. The lower process yield is due to the fact that only a certain portion can be crystallized out of a certain amount of feed. If, for example 50% of the initial mass is crystallized on the heat exchanger surface of the crystallizer and such process is repeated twice, the final product is only 25% of the initial amount of feed. The various intermediate fractions can also be subjected to further layer crystallization processes thereby increasing the yield but requiring an even more complex process structure with various purity - and recovery stages. The frequent repetition of multiple batch wise process steps with intermediate melting and re-crystallization consumes much more energy than a single stage process. Due to the much longer processing time along with intermediate periods at higher temperatures, the batchwise layer crystallization process is also subject to a higher dimerization risk.

Ruemekorf and Scholz in *"Crystal Clear Separation"* Hydrocarbon Engineering, June 2004, describe the use of suspension crystallization and wash column separation for the production of > 99.9 wt% 4,4' MDI product. Koole *et al.* also illustrated the potential of suspension based melt crystallization when applied to MDI in various presentations including Koole and Goncalves, "continuous melt crystallization, of MDI isomers", Netherlands Process Technology Symposium (NPS5), October 2005 and Zijl and Koole, "continuous suspension crystallization, for the separation of MDI isomers", Netherlands Process Technology Symposium (NPS8), October 2008. All such presentations focus on the production of Pure MDI and indicate that the respective process design is steered towards this direction.

US-B-6 271 414 (2001) describes the use of suspension crystallization and proposes remelting of about 30 wt% of the produced crystals and simultaneously increasing the temperature of the crystal slurry prior to introducing such slurry into a wash column for the production of Pure MDI of at least 99 wt%. This method is disadvantageous because it would require a substantially larger crystallizer when compared to a process without melting and therefore considerably adds to the investment and operational cost of the process.

Known crystallization processes for the purification of isocyanates from the prior art have the disadvantage of focusing on the production of only one ultrapure product and are inefficient in the sense that their purification potential is limited; typically multiple step processes must be executed in series to remove all the impurities. They also generate separate waste streams or use solvents that must be treated and or recovered separately and as a result are prohibitively expensive in terms of process investment and operating cost.

### Brief summary of the present Invention

In general terms, the present invention is accordingly based on mitigating or overcoming the disadvantages arising from the prior art.

In particular, the invention provides an efficient and economically viable method for the simultaneous productions of two isocyanate products, which method does not require any remelting and does not require frequent repetition of multiple batch wise process steps.

In addition, the invention provides a method which reduces exposure of the isocyanates to high operating temperatures and or alternating heating and cooling modes of operation to the maximum extent possible, thereby minimizing unwanted dimerization reactions and thus maximizing the product quality with high color stability and high yields.

Thus the present invention is directed to a method based on the following elements.
1. A suspension based melt crystallization process which is preferably operated in a continuous mode, wherein the crystals are grown in a carefully undercooled melt, such that spontaneous nucleation is minimized, in particular does not substantially occur, and in such a way that the crystals (i) have a very low content of impurities, in particular do not include any substantial impurities, and (ii) are suitable for the subsequent solid-liquid separation which can be carried out by known separation equipment, preferably in a wash column.
2. Control of the various product compositions discharged from the process is carried in an automated manner and fully adjustable and highly suitable to meet the demands required in large scale industry use.
3. Separation of the produced isocyanate crystals can be completed by well known solid-liquid separation unit operations, and is preferably carried out in a forced transport type wash column, which provides a highly efficient countercurrent washing operation without the loss of any wash liquid. In such a wash column the wash liquid comprises the melt of crystals as purified in said wash column.

Surprisingly it has been found that the method of the present invention can be used to produce most industrially relevant MDI product mixtures in a single process without any additionally required blending or recycle.

In one aspect, the present invention is directed to a method for separating an MDI feed stream, which feed stream comprises 4,4' MDI and one or more other MDI isomers, in particular 2,4' and/or 2,2' MDI, into a pure MDI product stream, which pure MDI product stream comprises 95-99 wt% 4,4' MDI, and into a technical grade MDI product stream, which technical grade MDI product stream is depleted in the 4,4' MDI and consequently enriched in the other MDI isomers, which method comprises the steps of:
a. cooling said MDI feed stream below its freezing point to form 4,4' MDI crystals, resulting in a mixed phase slurry of mother liquor and said crystals;
b. cooling said slurry to form an under-cooled melt, by which said crystals further grow, wherein the available surface for crystal growth is not less than 5000 m² per m³ of crystallizer volume;
c. separating said crystals from the mother liquid in a forced transport wash column;
d. heating at least a portion of the 4,4' MDI crystals to form a stream of liquid purified 4,4' MDI product; and
e. washing the 4,4' MDI crystals in the wash column with a washing stream, which washing stream comprises at least a portion of said liquid purified 4,4' MDI product, in a countercurrent washing operation;
   wherein the concentration of said technical grade MDI product stream is controlled by adopting the discharge quantity of said technical grade MDI, and wherein the purity of the pure MDI product stream is controlled by adjustment of the flow rate of said washing stream in said wash column.

Preferably step b, which involves further growing of the crystals, is carried out by holding the slurry in said crystallizer or in a separate container, so that a crystal residence time is obtained, which residence time is preferably between 15 minutes and 2 hours and more preferably between 30 minutes and 1 hour.

The crystal content of the slurry in step b is preferably between 15% and 50% by weight of said 4,4' MDI crystals and more preferably between 25% and 35%.

### Detailed description of the invention

In principle the novel method of this invention is suitable for the separation of all organic isocyanate mixtures as mentioned herein. All concentration figures and temperatures as further presented in the present application are given for the separation of 4,4' MDI rich feeds, but may easily be adopted by a person skilled in the art to the separation of the other organic isocyanates.

Frequently an industrial 4,4' MDI rich feedstock contains between 80% and 90% of 4,4' - MDI by weight (all percentages used herein are by weight, unless indicated otherwise) although solutions with as little as 75 wt% and up to 96 wt% can still benefit from the invention. The remainder MDI solution (other than the 4,4' MDI isomer) includes mostly the 2,4' MDI isomer and to lesser extent the 2,2' MDI isomer. Other minor impurities may include solvent residues, phenyl isocyanate derivatives, and other condensation products with more than two aromatic rings. The exact composition depends on the specific production method and is not particularly relevant for the present invention. In particular the 4,4' MDI and 2,4' MDI content are the most important parameters with respect to compositions of industrially relevant solutions.

Suspension based crystallization typically provides for continuous operation. While such continuous operation is preferred, the process of the present invention can also be operated batch wise without significant loss of efficiency. The large crystal mass of the suspended crystals provides a massive growth surface and allows for very slow and near ideal growth rates. It is preferred that the total available surface area for crystal growth in the suspension process of the present invention is approximately 5000-20000 m² per m³ of the crystallizer volume, more preferably 6000-15000 m²/m³, even more preferably 7000-12000 m²/m³. This far exceeds the growth surface typically available in layer type system which is generally limited to <100m² of crystal growth surface per m³ of system volume. The large growth surface in suspension based crystallization allows that any given undercooling is "absorbed" by such large growth surface and in turn results in extremely slow crystal growth rates in the range of 10⁻¹⁰ to 10⁻⁸ m/s. It was found that at these slow growth rates the isocyanate molecules can be included into the highly ordered crystal lattice under equilibrium conditions allowing new molecules sufficient time for the required diffusion from the homogeneously mixed bulk through the boundary layer before they get incorporated into the crystal structure.

In contrast to layer crystallization process, where the crystal growth rates typically exceeds the diffusion rate of the impurities, these impurities therefore get concentrated in front of the growing crystal layer. Such a situation typically leads to a dynamic, non-equilibrium controlled inclusion of impurities into the crystal lattice at such faster growth rates: the crystals grow in a dendrite like structure with inclusions of the high purity containing liquid close to the growth surface.

In the preparation of the isocyanate crystal suspension required according to the invention it is highly preferred that the crystals are grown in the undercooled melts at said slow growth rates. This can be accomplished in cooled stirred vessel crystallizers without scraping, or in scraped surface crystallizers or in disc crystallizer as described by Arkenbout in his review of commercially available crystallizers, see Arkenbout, "Melt Crystallization Technology", Technomic, 1995, pp 265-271. The latter two crystallizers have to be operated in a way to constantly sweep the heat exchanger surface in order to slightly undercool the bulk melt, but preventing a crystal layer to develop on the heat exchanger surface where higher undercooling may be present. Very generally, all suspension crystallizers which are mentioned in the above publication or which are listed in the present application are suitable to be used in accordance with the present invention for the preparation of the isocyanate suspension.

Suspension crystallization processes such as the crystallization of 4,4' MDI rich feeds involve both nucleation and crystal growth. Such two mechanisms are a function of the undercooling as well as other process parameters and system properties and finally determine the crystal size along with its distribution obtained with such crystallization processes. Two different types of nucleation are generally distinguished: primary or spontaneous nucleation, characterized when the undercooling exceeds a certain allowed figure, and secondary nucleation, which is a function of the crystal/crystal or crystal/hardware contact.

In a crystallization process at equilibrium state, the rate of newly formed nuclei must balance the number of crystals withdrawn from the system. The heat exchanger walls as required for the cooling in suspension based melt crystallization are typically scraped to prevent accumulation of the crystals in a crystal layer at the wall. The present inventors were surprised to find that scraping is not a requirement and that the process can be performed with heat exchangers without any scraping device as well. When sufficient mixing is applied, the driving temperature difference between the cooling medium and the crystal suspension for a process without scraped surface heat exchangers has to be limited to a figure of less than 5 °C. In case of insufficient turbulence this driving temperature difference must be lower. The inventors found that seeding is in most cases required to allow for a stable operation of a cooled vessel crystallizer without scraping. The present inventors believe that an insufficient nucleation rate in the crystallizer under such conditions is the reason for such phenomenon. When the rate at which crystals are withdrawn from the system is larger than the quantity of newly formed nuclei, the number of growing crystals in the vessel will gradually decrease. This in turn will eventually result in a sudden nucleation. While the effect is less pronounced than at the start of crystallization, it may lead to a sudden decrease in the crystal size with related problems in the subsequent solid-liquid separation. The present inventors found that seed crystals in an amount of preferably 0.01% to 2 % by weight and more preferably between 0.1% and 1% by weight are advantageously added to the mixing vessel in order to avoid cycling of the crystal size and allow for stable long operation periods. Such seeds can be added continuously or intermittently preferably at intervals between 30 and 60 minutes without any significant influence on the crystal product quality.

While scraping of the heat exchanger surface is not required, it was unexpectedly found that scraping is an efficient means to avoid the continuous or intermittent addition of seed crystals. The combined cooling and scraping action at the crystallizer wall produces sufficient fines (*i.e.* newly formed nuclei) as to avoid such addition of seed crystals. Since the production of such seed crystals as well as the continuous or intermittent addition thereof is a complex and costly procedure it is a preferred embodiment of this invention to use a crystallizer with a scraped surface heat exchanger. Such a scraped surface heat exchanger can also be operated at a higher temperature difference between the product suspension and the cooling side, thereby requiring less heat exchanger surface to be installed and thus reducing the cost of the system.

The required amount of energy to be discharged from the system to effect the crystallization can be affected by indirect cooling (*e.g.* jacketed vessels and heat exchangers by use of a coolant with or without phase transition of such coolant). Direct cooling (*e.g.* evaporation of a suitable coolant from the solution) may be possible but would require investigation to determine a suitable component. Water is frequently used in such direct cooled system by evaporating the water from the solution under vacuum. However, water must be completely avoided as this reacts with isocyanates exothermally, which can lead to excessive heat formation and possibly even explosion.

Very generally, the crystal suspension as produced according to the method of this invention can be separated by any solid-liquid device as known in the prior art. Such solid-liquid devices may include, for example, filter, vacuum filter, pressure filter, centrifuges and the like. The large crystal surface area allowing the optimum growth conditions during preparation of the MDI suspension, will negatively affect most attempts at separation. Impurities in the remaining liquid will adhere to all crystal surfaces and will be included into the interstitial spaces of a compacted crystal cake: a highly efficient solid-liquid separation device is therefore highly preferred to completely remove these impurities. Conventional mechanical separations require extensive washing to achieve even moderately pure product. In case molten product is used as washing liquid, about 10% to 20% of the crystallized product is lost and needs to be recycled to the crystallization process. This recycle requires larger equipment and adds to the heating/cooling cycle which will increase the production of dimers. In case a solvent is used as washing agent, such solvent would typically need to be recovered, which further increases complexity of the whole process.

In a specially preferred embodiment of the present invention the crystal suspension is therefore separated and further purified in wash columns. According to the invention all wash columns which are mentioned in the prior art referred to herein can be used. All prior art documents cited in the present application are incorporated herein by reference. The publications Melt Crystallazation - Fundamentals, Equipment and Applications edited by Joachim Ulrich, Shaker Verlag, 2003 and Melt Crystallization Technology, G.F. Arkenbout; Technomic, 1995 are mentioned by way of example. Wash columns with forced transport as described in EP-A-1 427 502, EP-A-0 920 894, EP-A-0 097 405, US-A-3 872 009 and EP-A-0 083 463 are particularly advantageous according to the invention and are also included herein by reference.

The equipment for the method of this invention can be made of glass and various types of plastics or metals or combination thereof, but preferably the system is made of standard available stainless steel.

While tracing is usually essential to keep all equipment required for performing the method of the present invention free from encrustations and/or blockages, the inventors found that a carefully designed and installed tracing system along with a proper control of the tracing temperature is very important for the achievement of the purification results of the present invention. Specifically, the total heat input supplied by the installed tracing must be minimized and the local temperature reached at the process surfaces must be monitored and maintained below a maximum temperature. Thus the temperature of the equipment surface preferably does not exceed 70 °C and is preferably below 55 °C. The inventors found that it is particularly useful to apply different tracing temperatures within the process and specifically at the unwashed part of the wash column and at the opposite washed part of the wash column.

Of course the novel procedure can also be used multiple times in series. When required, it is particularly advantageous to subject the residual mother liquor from the purity crystallizer to a second crystallization step where more product crystals are formed and to partially separate these crystals to form an enriched product that is combined with the initial starting mixture. In this way the recovery of the total process can be further increased, while potentially maintaining the option to crystallize the product only once. Such a combined process is disclosed in EP-A-1 398 064, which is also included herein by reference.

The invention will now be explained in greater detail herein below with reference to the figures.

The suspension based melt crystallization system typically comprises up to four main components:
(a) mixing vessel to suspend the crystals in mother liquor,
(b) heat exchanger, preferably with scraping device to discharge the amount of heat from the process as required for the formation of the crystals
(c) optionally an additional mixing vessel to optimize the mixed crystal flow, and
(d) a solid-liquid separation device, preferably a wash column, for efficient removal of the crystals from the mother liquor.

Components (a) and (b) may be combined into a single apparatus without any detrimental effects to the crystallization process.

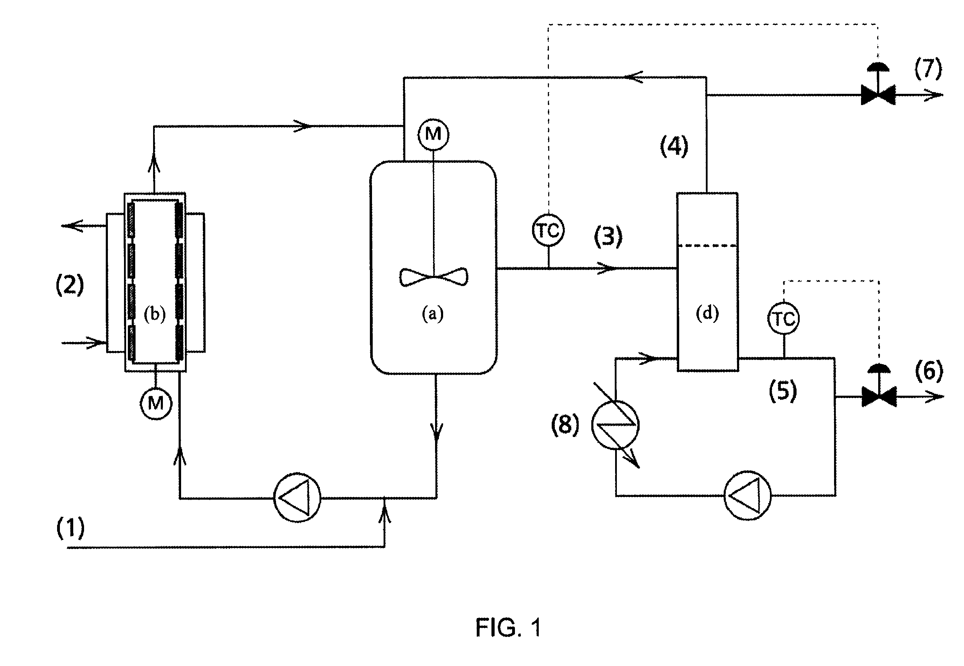

One specific embodiment of a process according to the present invention is described with reference to figure 1. Figure 1 illustrates that the 4,4' MDI rich feedstock (1) is fed to and circulated over the scraped surface heat exchanger crystallizer(s) (b) where coolant is circulated in the outer jacket (2).

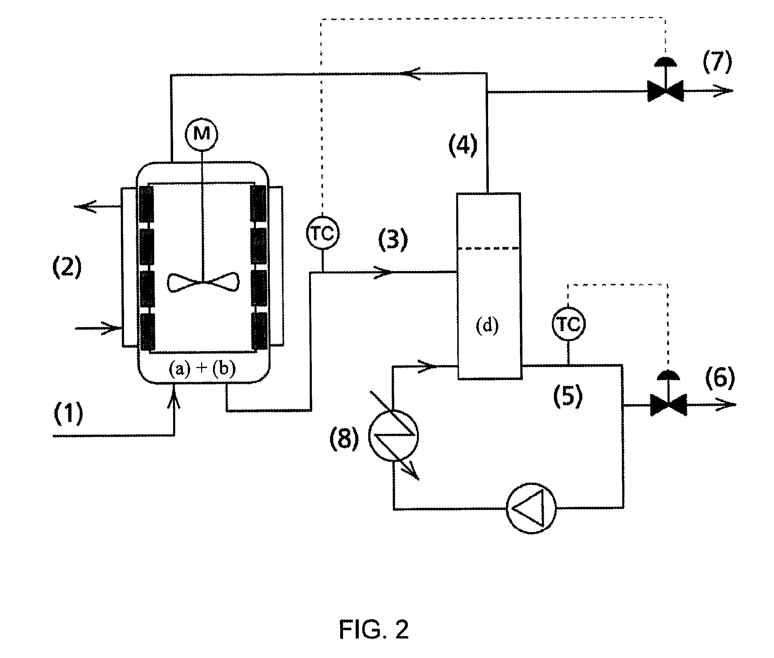

Figure 2 illustrates an equally advantageous configuration where components (a) and (b) are combined. The specific crystallization apparatus is readily available in industry.

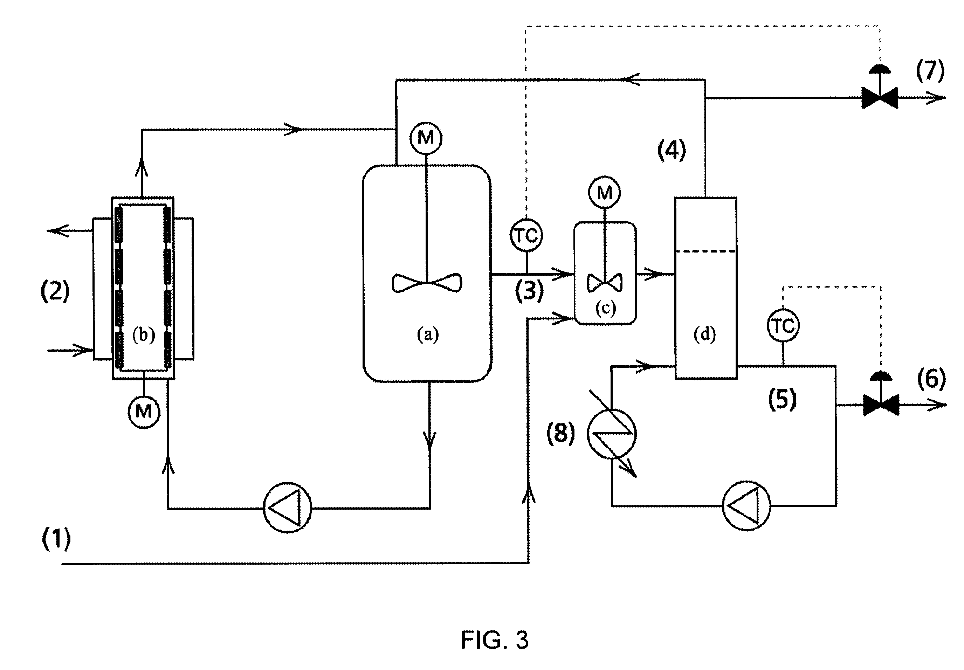

Figure 3 illustrates the process with the additional mixing vessel (c) mentioned above when related to the configuration described in figure 1. In this configuration, with optional mixing vessel (c), feed (1) enters at (c) instead of (b) and is returned to the crystallizer(s) via the filtrate in line (4). The optional mixing vessel can also be applied when the mixing vessel and crystallizer are combined. This configuration is not shown as it should be obvious to the skilled individual.

The scraped surface crystallizer(s) comprises a jacketed vessel containing a rotating scraper assembly to continuously remove encrustations from the cooled wall surface. The scraped surface crystallizer(s) are cooled by a secondary heat transfer liquid like *e.g.* water, brine, ethylene glycol solution or an evaporating refrigerant such as ammonia or Freon® in the outer jacket. Due to the freezing point of 4,4' MDI rich solutions, water can beneficially be used as cooling agent. The exact configuration is not particularly critical and suitable systems are readily available in industry. The process heat can be removed from the system by employing commonly known refrigeration systems. The process equilibrium temperature in the scraped surface crystallizer(s) will typically range from +15 °C to +30 °C and preferably +18 °C to +23 °C. The mixing vessel(s) (a) are used to suspend the crystal slurry with typically 15% to 50% crystals by weight and preferably 25% to 35% crystals by weight in the mother liquor.

This amount of crystals provides a very large surface area for crystal growth. High growth surface areas result in very low growth rates. The very slow growth rates provided by suspension based crystallization ensure that the impurities that would normally be included in the rapidly growing crystal surface of a layer crystallization process are now excluded and remain in the mother liquor and results in pure product crystals.

Surprisingly the intensity of the mixing is not critical and the mixing element can be maintained at a very low rotational speed of 40 to 100 rpm or just enough to keep the crystal mass from settling to the bottom of the vessel. Suitable mixing vessel apparatus is readily available in industry.

The crystal volume in the mixing vessel should be sufficient to provide an average crystal residence time in the range of 15 minutes up to 2 hours and preferably in the range of 30 minutes to 1 hour. Preferably the residence time does not extend beyond 1 hour since such longer periods will only marginally increase the crystal size. Excessively long residence times increase the complexity and cost of the required equipment. From the mixing vessel(s) the pure crystals suspended in impure mother liquor (*i.e.* slurry) are fed to one or more wash column(s) (d) via supply line (3). The wash column separates the crystals by compressing the slurry and allowing most of the mother liquor to leave through a filter via discharge line (4) to produce a thickened slurry in the form of a compact crystal bed with preferably between 60% and 90% crystals by weight and more preferably 70% to 80% by weight with the remainder being liquid mother liquor containing essentially all of the impurities. The compacted crystal bed is mechanically forced through the column by hydraulic or other mechanical force. The crystal bed is disintegrated and reslurried opposite the original slurry entry point to form pumpable new slurry in the melter loop (5).

The compact bed is composed of billions of individual crystals and thus forms a porous crystal cake mass within the wash column cylinder. The pressure in the melt loop is controlled by the amount of product discharged in stream (6). The pressure in the melt loop (5) is thus controlled to a value higher than in the mother liquor discharge line (4). The open space between the solid crystals is initially filled with impure mother liquor. Due to this pressure difference generated over the compacted crystal bed the melted product is forced counter-current to the crystal flow (*p*(5) > *p*(4)). The wash column systems typically use low pressure steam to melt the crystals in a heat exchanger (8) before discharging the purified product (6). In practice this can be any commercially available type heater including electric, hot oil, hot air or even heat exchangers type systems using the hot feed stock.

The crystals enter the wash column through line (3) at substantially the same temperature as the crystal slurry found in the mixing vessel (a). For MDI mixtures this is typically in the range from +15 °C to +30 °C and preferably from +18 °C to +23 °C. The exact temperature will depend on the specific impurities found in the original 4,4' MDI rich feed. The purified 4,4' MDI product has a melting temperature of typically +40 °C. As the warm purified product flows through the porous crystal bed it will contact the colder crystals and the purified product will re-crystallize onto the surface of the colder crystals. This recrystallization produces new crystalline product from the wash liquid. These new crystals are then carried out with the original crystal mass thus preventing the loss of valuable pure product to the process. The impurities can effectively be washed from the surface and then mostly remain with the mother liquor. Surprisingly, it has been found that the wash column can also be operated in such a way as to allow a certain portion of the mother liquor to remain in the crystal bed. Unlike most other solid-liquid separation devices where the temperature of the wash liquid needs to be controlled very carefully in order to allow for stable washing operation to avoid (i) direct crystallization of the wash liquid on the crystal cake in case of a too cold wash liquid temperature or (ii) melting of the crystal cake in case of a too warm wash liquid temperature — and which devices are therefore not very suitable for a proper control of the washing operation — , a wash column — due to its intrinsic wash liquid temperature control inside the column — allows for a stable and consistent washing process with an adjustable washing efficiency: When the washing action in a wash column is complete, *i.e.* total removal of the mother liquor, this washing action is 100% efficient. The washing efficiency as used herein may be defined as the amount of mother liquor that is removed from the compressed crystal bed. With 100% washing efficiency all of the mother liquor is removed from the crystal mass (*i.e.* all of mother liquor has been replaced with pure wash liquid) and 80% washing efficiency means that 20% of the crystal mass is still mother liquor. When the washing allows some residual mother liquor (*e.g.* 2% residual mother liquor or 98% efficient) this residual mother liquor contains impurities that will end up in the pure product and will reduce the final purity of the product. In many cases the product purity required by the end user is less than 99.9 wt% 4,4' MDI and by reducing the washing efficiency to a specified value a pure product with less than 99.9% 4,4' MDI can be produced. Thus the final product purity can easily be adjusted by the process of the present invention.

The purity of the product discharged can be observed by numerous methods including in-line or on-line measurement techniques of gas chromatography, spectrophotomeric or infrared and near infrared techniques. These methods generally require expensive equipment, frequent calibration and maintenance. The approximate purity, suitable for operation and control of the present method can be easily determined through simple and inexpensive temperature measurement equipment. When the solid crystals from the disintegrated bed are mixed with the melted crystals discharged from the heat exchanger (8) the resulting slurry will reach an equilibrium temperature determined by the composition of the 4,4' MDI. As this liquid is mostly 4,4' MDI (>95 wt%) the specific other impurities are not significant. After an initial calibration of the temperature measurement the temperature of the slurry as it leaves the wash column can be used to indicate the purity of this stream and the composition of the discharged product (6) can be related now directly to the temperature at (5). This temperature (*i.e.* product purity) can be used to control the washing action in the wash column by adjustment of the discharged product at (6). In this way the column can be automatically controlled to give a range of product purities. In practice this range is limited to the purity resulting from a washing efficiency between 80% and 100%. Since the temperature of the slurry at (3) is the equilibrium temperature of the liquid discharged at (7). The specific mother liquor impurity level in the liquid discharged at (7) can be related to the temperature in the same manner as the purified product. In this simple manner the composition of both products can be automatically controlled to provide a range of useful compositions from the original feed stock. When this washing efficiency is coupled with a specific mother liquor impurity level the range of products achieved using this method is increased and the simultaneous production of both products can be tailored to meet most industrially relevant combinations in a single process step.

By using the present invention a given feed can be split into two product streams (referred to as "pure" and "technical grade" herein). Possible combinations of pure and technical grade are determined mainly by the limitations in the washing efficiency. Very suitable combinations of pure and technical grade product streams that both find demand in industry are discussed below.

In one preferred embodiment, the process of the invention is used to provide a pure MDI product comprising 85-90 wt.% 4,4' MDI in combination with a technical grade MDI composition comprising 47-52 wt.% 4,4' MDI. This combination is based on an industrially relevant reject (*viz*. technical grade) stream, 47-52% is the eutectic composition (which is typically the lower limit for all crystallization processes) and the 85-90% corresponds to the best pure product to be obtained under those conditions.

In another preferred embodiment, a pure MDI product comprising 96-99 wt.% 4,4' MDI in combination with a technical grade MDI composition comprising 55-65 wt.% 4,4' MDI is provided. This is based on the best reject that can be obtained when making the normal expected "pure" product. Typical requests for pure product is 98-98.5 wt.%, which is thus even more preferred.

In yet another preferred embodiment, a pure MDI product comprising 98.5-99.5 wt.% 4,4' MDI in combination with a technical grade MDI composition comprising 65-75 wt.% 4,4' MDI is provided, which is based on the best reject that can be made in accordance with the invention when making high purity product.

These and other compositions can be obtained by controlling the reject composition and washing efficiency as detailed hereinabove and in the examples given below. In this way two product streams can be obtained. In general, the composition of the mother liquor and the amount left in the crystal bed will produce a specific "pure" product. The amount of mother liquor left in the crystal bed is controlled by the wash efficiency and the composition of the mother liquor is controlled by technical grade MDI outlet valve.

Figure 4 shows an "operation envelope" which indicates product combinations that can be obtained in accordance with the present invention depending on the requirements of the end user.

Figure 5 shows the equilibrium temperature diagram for the binary mixture 4,4' MDI and 2,4' MDI.

The invention will be illustrated in more detail in the examples below, which shall not be construed as limiting to the present invention. The following examples are based on operation of a crystallization system comparable to the process described in Figure 2 including a piston type wash column as solid-liquid separation device. The feed stock is based on a binary mixture of purified 4,4' MDI and 2,4' MDI. The equilibrium temperature of industrial products may vary slightly from these examples due to the presence of other impurities (*e.g.* 2,2' MDI). These other compositions were not studied as they will not significantly affect the results except for the temperature measurement.

### Example 1

Slurry temperature +21 °C washing efficiency 92%. Based on an industrially relevant composition for Technical Grade MDI of 50 wt% 4,4' MDI. The crystallizer was operated so as to achieve an equilibrium temperature of 21 °C and crystal residence time of 20 minutes. The maximum washing efficiency achieved during the operation was 92% which corresponds to a product purity of 96 wt% 4,4' MDI with resulting temperature at (5) of 39.2 °C. When the washing efficiency is reduced by discharging increasing amounts of the pure MDI and maintaining the same Technical Grade MDI (*i.e.* 20 °C in the crystallizer) the temperature at (5) was 37.3 °C which corresponds to a Pure MDI composition of approximately 90 wt% 4,4' MDI.

### Example 2

Slurry temperature +18 °C washing efficiency 80%. The maximum recovery of a crystallization system is based on discharge of the eutectic composition. Based on the binary system, when the crystallizer is operated at this composition, both components will crystallize to form a crystal mass and mother liquor, with identical compositions. No further separation is possible, using only crystallization, beyond this point. We therefore chose a target composition near the reported eutectic (42.5 wt% 4,4' MDI and 17 °C). The crystal growth near the eutectic is much slower and the crystal residence time was increased to 45 minutes, double the previous example, Under these conditions the resulting separation was more difficult and the maximum washing efficiency was only 80% which resulted in a temperature at (5) of only 37 °C (*i.e.* 88.9 wt% 4,4' MDI). This represents the maximum composition of Pure MDI possible under these conditions.

### Example 3

Slurry temperature +25 °C washing efficiency 96%. Higher compositions of Pure MDI are interesting due to the polymer requirements of the various high performance applications. As an example case we chose to examine a Pure MDI with about 98.5 wt% 4,4' MDI. Based on product temperature at (5) of 40 °C and a crystal residence time of 20 minutes the corresponding Technical Grade MDI was found at 25 °C (*i.e.* 58 wt% 4,4' MDI) and represents a washing efficiency of 96%. Maintaining this Technical Grade MDI temperature of 25 °C the lowest Pure MDI composition was produced by product temperature at (5) of 37.9 °C (*i.e.* about 92 wt% 4,4' MDI) which corresponds to about 80% washing efficiency.

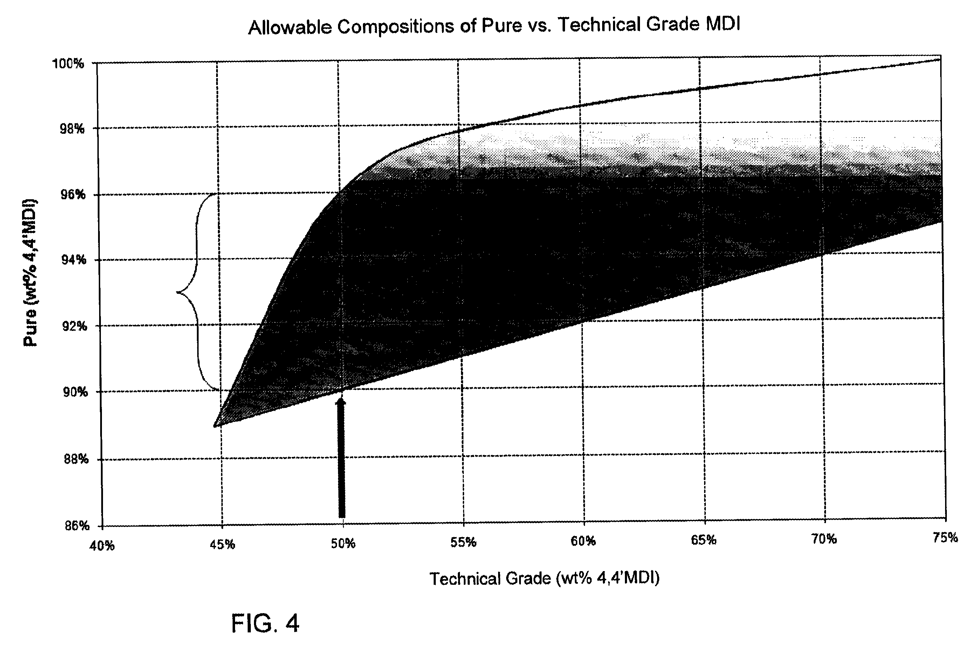

Based on these results a general efficiency curve can be calculated for the equipment configuration described and the binary system of 4,4' and 2,4' MDI. This results in the operation envelope shown in figure 4, where multiple product combinations are possible depending on the requirements of the end user. The process can be automatically controlled by adjusting the corresponding Technical Grade and Pure product outlet equilibrium temperatures to the respective values within the operating envelope illustrated in figure 4.

As illustration of this principle: the production of a Technical Grade MDI of about 50 wt% 4,4'MDI can be completed in combination with a Pure MDI product anywhere between 90wt% and 96wt% 4,4' MDI. Start with 50wt% 4,4'MDI on the x-axis and then any Pure composition within the operation envelope can be found by moving upward along the 50wt% line.

## Claims

1. Method for separating an MDI: (methylene diphenyl diisocyanate) feed stream, which feed stream comprises 4,4' MDI and one or more other MDI isomers, in particular 2,4' and/or 2,2' MDI, into a pure MDI product stream, which pure MDI product stream comprises 95-99 wt% 4,4' MDI, and into a technical grade MDI product stream, which technical grade MDI product stream is enriched in said other MDI isomers, which method comprises the steps of:
a. cooling said MDI feed stream below its freezing point to form 4,4' MDI crystals, resulting in a mixed phase slurry of mother liquor and said crystals;
b. cooling said slurry to form an under-cooled melt, by which said crystals further grow, wherein the available surface for crystal growth is not less than 5000 m² per m³ of crystallizer volume;
c. separating said crystals from the mother liquid in a forced transport wash column;
d. heating at least a portion of the 4,4' MDI crystals to form a stream of liquid purified 4,4' MDI product; and
e. washing the 4,4' MDI crystals in the wash column with a washing stream, which washing stream comprises at least a portion of said liquid purified 4,4' MDI product, in a countercurrent washing operation;
wherein the concentration of said technical grade MDI product stream is controlled by adopting the discharge quantity of said technical grade MDI, and wherein the purity of the pure MDI product stream is controlled by adjustment of the flow rate of said washing stream in said wash column.

2. Method according to claim 1, wherein said adopting of the discharge quantity of said technical grade MDI comprises measuring the temperature in said crystallizer and controlling the temperature in said crystallizer at a fixed value by adjustment of the technical grade MDI outlet valve.

3. Method according to any of the previous claims, wherein said adjustment of the flow rate of said washing stream comprises a temperature control in the pure MDI washing stream, wherein the temperature of the pure MDI washing stream is maintained at a fixed value by adjustment of the pure MDI outlet valve, to obtain a washing efficiency of between 80% and 100%.

4. Method according to any of the previous claims, wherein said pure MDI product comprises 85 wt.%-90 wt.% 4,4' MDI, and wherein said technical grade MDI composition comprises 47 wt.%-52 wt.% 4,4' MDI.

5. Method according to any of the claims 1-3, wherein said pure MDI product comprises 96 wt.%-99 wt.% 4,4' MDI, and wherein said technical grade MDI composition comprises 55 wt.%-65 wt.% 4,4' MDI.

6. Method according to any of the claims 1-3, wherein said pure MDI product comprises 98.5 wt.%-99.5 wt.% 4,4' MDI, and wherein said technical grade MDI composition comprises 65 wt.%-75 wt.% 4,4' MDI.

## Patentansprüche

1. Verfahren zur Trennung eines MDI (Methylendiphenyl-Diisocyanat)-Zufuhrstroms, der 4,4' MDI und ein oder mehr MDI-Isomere, insbesondere 2,4' und/oder 2,2' MDI, umfasst, in einen reinen MDI-Produktstrom, der 95-99 Gew.-% 4,4' MDI umfasst, und in einen MDI-Produktstrom von technischer Qualität, der mit den anderen MDI-Isomeren angereichert ist, wobei das Verfahren die Schritte umfasst:
a. Kühlen des MDI-Zufuhrstroms unter seinen Gefrierpunkt, um 4,4' MDI-Kristalle zu bilden, resultierend in einer Mischphasenmasse aus Mutterlauge und den Kristallen;
b. Kühlen der Masse, um eine unterkühlte Schmelze zu bilden, wodurch die Kristalle weiter wachsen, wobei die verfügbare Oberfläche für das Kristallwachstum nicht weniger als 5000 m² pro m³ Kristallisatorvolumen beträgt;
c. Abtrennen der Kristalle aus der Mutterlauge in einer Zwangstransport-Waschsäule;
d. Erwärmen von mindestens einem Teil der 4,4' Kristalle, um einen Strom von flüssigem gereinigtem 4,4' MDI Produkt zu bilden; und
e. Waschen der 4,4' MDI-Kristalle in der Waschsäule mit einem Waschstrom, wobei der Waschstrom in einem Gegenstrom-Waschvorgang mindestens einen Teil des flüssigen gereinigten 4,4' MDI-Produkts enthält,;
wobei die Konzentration des Produktstroms des MDI von technischer Qualität durch Anpassung der Abflussmenge des MDI von technischer Qualität gesteuert wird, und wobei die Reinheit des reinen MDI-Produktstroms durch Anpassung der Flussrate des Waschstroms in der Waschsäule gesteuert wird.

2. Verfahren nach Anspruch 1, wobei die Anpassung der Abflussmenge des MDI von technischer Qualität das Messen der Temperatur in dem Kristallisator und die Steuerung der Temperatur in dem Kristallisator bei einem festen Wert durch Anpassung des Abflussventils des MDI von technischer Qualität umfasst.

3. Verfahren nach einem der vorhergehenden Ansprüche, wobei die Anpassung der Flussrate des Waschstroms eine Temperatursteuerung in dem reinen MDI-Waschstrom umfasst, wobei die Temperatur des reinen MDI-Waschstroms durch Anpassung des Ablassventils für reines MDI auf einem festen Wert gehalten wird, um eine Wascheffizienz zwischen 80 % und 100 % zu erhalten.

4. Verfahren nach einem der vorhergehenden Ansprüche, wobei das reine MDI-Produkt 85 Gew.-% - 90 Gew.-% 4,4' MDI umfasst, und wobei die MDI-Zusammensetzung von technischer Qualität 47 Gew.-% - 52 Gew.-% 4,4' MDI umfasst.

5. Verfahren nach einem der Ansprüche 1-3, wobei das reine MDI-Produkt 96 Gew.-% - 99 Gew.-% 4,4' MDI umfasst, und wobei die MDI-Zusammensetzung von technischer Qualität 55 Gew.-% - 65 Gew.-% 4,4' MDI umfasst.

6. Verfahren nach einem der Ansprüche 1-3, wobei das reine MDI-Produkt 98,5 Gew.-% - 99,5 Gew.-% 4,4' MDI umfasst, und wobei die MDI-Zusammensetzung von technischer Qualität 65 Gew.-% - 75 Gew.-% 4,4' MDI umfasst.

## Revendications

1. Procédé pour séparer un courant d'alimentation de MDI (méthylène diphényl diisocyanate), lequel courant d'alimentation comprend du 4,4'-MDI et un ou plusieurs autres isomères de MDI, en particulier du 2,4'-MDI et/ou du 2,2'-MDI, en un courant de produit MDI pur, lequel courant de produit MDI pur comprend 95 à 99% en poids de 4,4'-MDI, et en un courant de produit MDI de qualité technique, lequel courant de produit MDI de qualité technique est enrichi en lesdits autres isomères du MDI, ce procédé comprenant les étapes suivantes:
a. le refroidissement dudit courant d'alimentation de MDI en dessous de son point de congélation pour former des cristaux de 4,4'-MDI, donnant lieu à une suspension de phase mixte de la liqueur mère et desdits cristaux;
b. le refroidissement de ladite suspension pour former une masse fondue sous-refroidie, conduisant à la croissance supplémentaire desdits cristaux, la surface disponible pour la croissance des cristaux étant d'au moins 5000 m² par m³ de volume de cristallisoir;
c. la séparation desdits cristaux de la liqueur mère dans une colonne de lavage à transport forcé;
d. le chauffage d'au moins une partie des cristaux de 4,4'-MDI pour former un courant de produit 4,4'-MDI purifié liquide; et
e. le lavage dans une opération de lavage à contre-courant des cristaux de 4,4'-MDI dans la colonne de lavage par un courant de lavage, lequel courant de lavage comprend au moins une partie dudit produit 4,4'-MDI purifié liquide,
procédé dans lequel la concentration dudit courant de MDI de qualité technique produit est régulée par adoption de la quantité de MDI de qualité technique déchargée, et dans lequel la pureté du courant de produit MDI pur est régulée par ajustement du débit d'écoulement dudit courant de lavage dans ladite colonne de lavage.

2. Procédé selon la revendication 1, dans lequel ladite adoption de la quantité de MDI de qualité technique déchargée comprend la mesure de la température dans ledit cristallisoir et le contrôle de la température dans ledit cristallisoir à une valeur fixée par réglage de la soupape de sortie du MDI de qualité technique.

3. Procédé selon l'une quelconque des revendications précédentes, dans lequel ledit ajustement du débit d'écoulement dudit courant de lavage comprend le contrôle de la température du courant de lavage du MDI pur, dans lequel la température du courant de lavage du MDI pur est maintenue à une valeur fixe par réglage de la soupape de sortie du MDI pur, pour obtenir une efficacité de lavage comprise entre 80% et 100%.

4. Procédé selon l'une quelconque des revendications précédentes, dans lequel ledit produit MDI pur comprend 85 à 90% en poids de 4,4'-MDI, et dans lequel ladite composition de MDI de qualité technique comprend 47% à 52% en poids de 4,4'-MDI.

5. Procédé selon l'une quelconque des revendications 1 à 3, dans lequel ledit produit MDI pur comprend 96% à 99% en poids de 4,4'-MDI, et dans lequel ladite composition de MDI de qualité technique comprend 55% à 65% en poids de 4,4'-MDI.

6. Procédé selon l'une quelconque des revendications 1 à 3, dans lequel ledit produit MDI pur comprend 98,5 à 99,5% en poids de 4,4'-MDI, et dans lequel ladite composition de MDI de qualité technique comprend 65% à 75 en poids de 4,4'-MDI.
